# FASCICULE DE BREVET EUROPEEN

(11) **EP 0 758 260 B1**
(45) Date de publication et mention de la délivrance du brevet: **17.06.1998**
(21) Numéro de dépôt: 95918662.8
(22) Date de dépôt: 02.05.1995
(51) Int. Cl.: A61N 1/30

(54) **PROCEDE ET DISPOSITIF DE MESURE DE LA QUANTITE D'UN PRINCIPE ACTIF CONTENU DANS UN RESERVOIR**
VERFAHREN UND VORRICHTUNG ZUR MESSUNG DER MENGE EINES AKTIVEN BESTANDTEILS IN EINEM BEHÄLTER
METHOD AND DEVICE FOR MEASURING THE AMOUNT OF ACTIVE PRINCIPLE IN A CONTAINER

(30) Priorité: 06.05.1994 FR 9405633
(43) Date de publication de la demande: 19.02.1997
(73) Titulaire: LABORATOIRES D'HYGIENE ET DE DIETETIQUE L.H.D., 75008 Paris (FR)
(72) Inventeur: MILLOT, Philippe, F-21000 Dijon (FR); LIOTARD, Anne, F-21000 Dijon (FR)
(74) Mandataire: Colas, Jean-Pierre
(86) Numéro de dépôt international: FR9500568
(87) Numéro de publication internationale: WO9530450

(56) Documents cités:
- EP-A- 0 277 314
- EP-A- 0 555 510
- WO-A-90/03825
- FR-A- 2 562 800

## Description

La présente invention est relative à un procédé et à un dispositif de mesure de la quantité d'un principe actif contenu dans un réservoir, ainsi qu'à un réservoir conçu pour permettre une telle mesure. Plus particulièrement, l'invention est relative à de tels procédé, dispositif et réservoir mis en oeuvre dans le cadre d'une administration transdermique de médicaments assistée par ionophorèse.

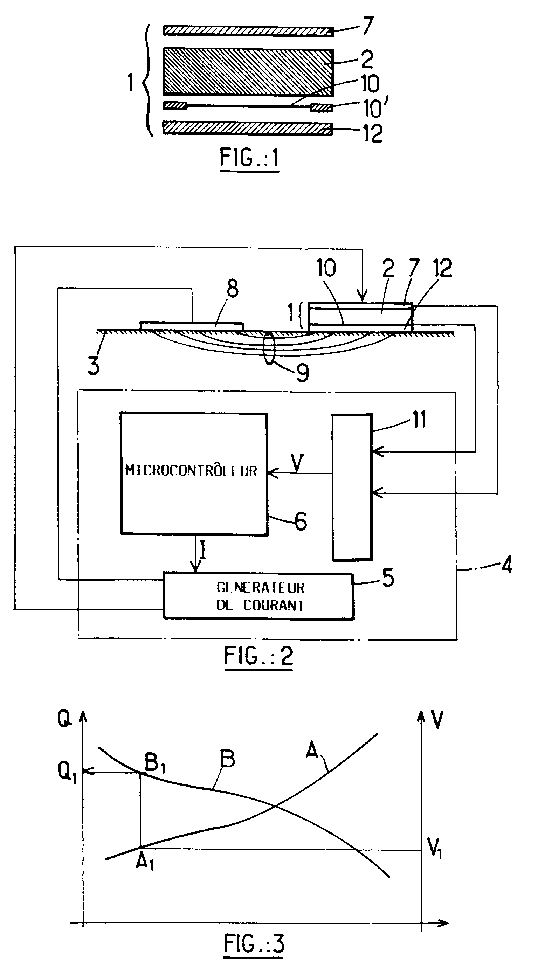

On a représenté schématiquement à la figure 2 du dessin annexé des moyens utilisables pour procéder à une telle administration. Ces moyens comprennent essentiellement, de manière connue, un réservoir 1 comprenant une couche 2 d'un matériau, tel qu'un polymère hydradatable ou "hydrogel", imprégné d'une solution du principe actif, cette couche 2 étant conçue pour être mise en contact avec la peau 3 d'un patient. Le principe actif est présent dans la solution sous une forme ionisée de manière que son passage à travers la peau du patient puisse être assisté par ionophorèse. Pour ce faire, le réservoir est connecté à un module électronique 4 comprenant des moyens de génération 5 et des moyens de commande 6 d'un courant dit "thérapeutique" qui passe dans le patient entre une électrode 7, qui peut faire partie du réservoir 1, et une électrode 8 adjacente de manière à établir des lignes de courant 9 sous la peau du patient. Les ions du principe actif descendent ces lignes de courant à partir du réservoir 1 pour traverser le derme et passer dans les vaisseaux capillaires sous-jacents. Les moyens de commande 6, un microcontrôleur par exemple, commandent l'amplitude et la forme d'onde du courant appliqué de manière que la quantité de principe actif passant dans le sang soit modulée dans le temps suivant un programme d'administration précis, établi à partir de considérations pharmacologiques.

Ce programme doit ainsi respecter des valeurs minimum et maximum des doses de principe actif appliquées par unité de temps. Une surdose peut être très dangereuse pour la sécurité du patient alors qu'une dose insuffisante ne permet pas d'assurer l'activité pharmacologique voulue. Il y a lieu de remarquer à cet égard que les quantités de principe actif à administrer varient bien entendu d'un principe actif à un autre et que le programme d'administration doit pouvoir s'adapter aux variations de la perméabilité de la peau, d'un patient à l'autre.

On comprend que pour assurer le suivi du programme, il est alors nécessaire que les moyens de commande soient asservis à une mesure de la quantité de principe actif effectivement administrée au patient, ceci tout au long de la durée de l'administration.

Pour ce faire, on propose dans la demande de brevet européen No. 0 277 314 au nom de R. TAPPER, un procédé et un appareil de dosimétrie des quantités de principe actif administrées, opérant par une intégration dans le temps du courant "thérapeutique" passant dans le patient entre l'électrode 7 et l'électrode 8. Ce procédé est grevé de l'inconvénient suivant lequel le courant passant entre les deux électrodes n'est pas forcément représentatif du nombre d'ions du principe actif qui sont sortis du réservoir pour passer dans le sang du patient. L'ionophorèse présente en effet un rendement inférieur à l'unité et, de surcroît, variable d'un patient à l'autre, d'un état de la peau du patient à un autre, cet état variant progressivement pendant l'administration du médicament, qui peut durer couramment plusieurs heures.

Une autre solution théoriquement envisageable, mais lourde, consisterait à doser in vivo la quantité de principe actif présente dans le sang du patient.

La présente invention a pour but de fournir un procédé et un dispositif de mesure de la quantité d'un principe actif contenu dans un réservoir, qui ne présente pas les inconvénients mentionnées ci-dessus de la technique antérieure.

La présente invention a aussi pour but de fournir un réservoir de principe actif convenant à la mise en oeuvre du procédé selon l'invention.

On atteint ces buts de l'invention, ainsi que d'autres qui apparaîtront à la lecture de la description qui va suivre, avec un procédé de mesure de la quantité d'un principe actif contenu sous une forme ionisée dans un réservoir constitué par une couche d'un matériau imprégné d'une solution de ce principe actif, remarquable en ce qu'on mesure la conductivité du matériau imprégné et on tire une mesure de la quantité de principe actif contenu dans le réservoir de cette mesure de conductivité. Comme on le verra plus loin, la conductivité ou la conductance du réservoir est fonction de la quantité de principe actif contenue dans ce réservoir. Par différence on peut en déduire la quantité de principe actif passée dans le sang d'un patient pendant une administration transdermique de médicaments.

Suivant une caractéristique du procédé selon l'invention, pour mesurer la conductivité du matériau imprégné contenu dans le réservoir, on fait passer un courant d'intensité prédéterminée dans celui-ci, et on mesure la tension électrique qui apparaît alors entre deux électrodes séparées par au moins une partie de la couche du matériau imprégné.

Pour la mise en oeuvre de ce procédé, l'invention fournit un dispositif comprenant a) des première et deuxième électrodes placées de part et d'autre et en contact électrique avec au moins une partie de la couche du matériau imprégné du réservoir, b) des moyens pour faire passer le courant électrique d'intensité prédéterminée dans le matériau imprégné et c) des moyens sensibles à la tension relevée entre les électrodes pour calculer la quantité de principe actif contenue dans le réservoir.

L'invention fournit encore un réservoir utilisable dans un tel dispositif, comportant une couche d'un matériau susceptible d'être imprégné d'une solution du principe actif en vue d'une administration transdermique de ce principe actif à un patient, à travers une face de cette couche appliquée contre la peau du patient, ce réservoir étant remarquable en ce qu'il comprend une électrode perméable à la solution de principe actif, appliquée sur ladite surface de la couche du matériau imprégnable.

D'autres caractéristiques et avantages de la présente invention apparaîtront à la lecture de la description qui va suivre et à l'examen du dessin annexé dans lequel :
- la figure 1 est une représentation schématique d'un réservoir suivant la présente invention, en coupe transversale éclatée,
- la figure 2 est une représentation schématique d'un dispositif pour la mise en oeuvre du procédé selon l'invention, déjà partiellement décrit dans le préambule de la présente description, et
- la figure 3 est une abaque utilisée dans le procédé de mesure suivant l'invention.

On se réfère à la figure 1 où il apparaît que le réservoir 1 suivant l'invention comprend, outre la couche 2 d'hydrogel imprégnée d'une solution de principe actif sous forme ionisée et la première électrode 7 assurant l'application du courant thérapeutique, une deuxième électrode 10, de mesure, montée par exemple sur un cadre 10' et placée sur la face de la couche 2 qui est opposée à celle plaquée contre la première électrode 7. Avantageusement, le réservoir 1 comprend encore une couche 12 d'hydrogel neutre déposée sur l'électrode 10 de manière à venir en contact avec la peau d'un patient lors d'une administration transdermique de médicaments assistée par ionophorèse, pour des buts que l'on expliquera plus loin.

On notera incidemment que la première électrode 7 peut, en variante, être solidaire du module électronique 4, la couche d'hydrogel 2 du réservoir étant mise en contact avec cette électrode juste avant une administration de médicament, par exemple.

Suivant l'invention, l'électrode 10 de mesure est perméable à la solution de principe actif de manière que celle-ci puisse venir mouiller la peau du patient. A titre d'exemple seulement, cette électrode peut être constituée par une grille en fil métallique fin, au pas de 1 fil/2 mm ou de 0,5 fil/2 mm par exemple.

Par ailleurs, le dispositif représenté à la figure 2 comprend des moyens 11 alimentés par la différence de potentiel prélevée entre les électrodes 7 et 10 pendant le passage du courant thérapeutique établi par le générateur 5, ces moyens 11 délivrant au microcontrôleur un signal de tension V image de ladite différence de potentiel. Ce signal subit dans le microcontrôleur 6 une conversion analogique/numérique en vue de calculs ultérieurs. Les moyens 11 constituent un étage d'adaptation qui peut être réalisé classiquement à l'aide d'un amplificateur différentiel, par exemple.

Comme on l'a vu plus haut, le microcontrôleur 6 commande l'amplitude, la forme d'onde etc... du courant délivré par le générateur 5. Ainsi, le microcontrôleur connaît-il exactement, à l'instant où il relève la tension V, le courant I qui traverse l'électrode 7 et la couche d'hydrogel imprégnée de principe actif.

De ces deux paramètres, on peut déduire la conductivité globale de la couche (2) d'hydrogel, conductivité qui est fonction de la quantié d'ions du principe actif présents dans la couche 2 au moment de la mesure de I et de V. Si les seuls ions présents dans la couche d'hydrogel sont ceux du principe actif, on peut déduire la quantité de principe actif présente dans la couche de la seule mesure de conductivité de la couche.

En fait, cependant, la couche d'hydrogel contient souvent d'autres espèces ionisées. Il en est ainsi notamment lorsque, classiquement, le principe actif est introduit dans la couche d'hydrogel par l'intermédiaire d'une solution saline, de chlorure de sodium par exemple. Si le principe actif est présent dans la solution sous la forme d'ions de charge positive, il est clair que les co-ions Na⁺ de la solution influencent, en fonction de leur concentration et leur mobilité, la conductivité globale de la couche 2. De même si l'électrode 7 est en argent/chlorure d'argent, on peut trouver dans la couche 2 des ions Ag⁺ qui affectent sa conductivité.

En pratique, suivant l'invention, on tient compte de tous les ions susceptibles d'affecter la conductivité de la couche (2), autres que ceux du principe actif, en procédant à des opérations d'étalonnage préalables, basées sur des mesures réalisées in vitro et/ou in vivo, ces opérations permettant d'établir l'abaque illustrée par la figure 3, qui permet de tirer la quantité de principe actif contenu dans la couche 2 de la seule mesure de la tension V entre les électrodes 7 et 10.

Pour tracer cette abaque, on peut partir d'une cellule de mesure "in vitro" comprenant une enceinte pour un milieu receveur des ions d'un principe actif ayant traversé un échantillon de peau accolé au réservoir de la figure 1, sous assistance ionophorétique. On dose alors périodiquement la concentration en principe actif du milieu receveur et on relève la tension V observée alors entre les électrodes 7 et 10 (graphe A). Des mesures de concentration on tire par différence, le graphe B qui illustre la décroissance de la quantité Q de principe actif demeurant dans le réservoir, en fonction du temps.

Cette abaque, une fois dressée, est utilisée de la manière suivante : lors d'une administration transdermique d'un principe actif donné dans une solution donnée de concentration de départ donnée, on mesure à un certain instant une tension V₁ entre les électrodes 7 et 10. Cette tension permet de déterminer sur le graphe A un point A₁ d'ordonnée V₁ et ensuite un point B₁ sur le graphe B, ayant l'abscisse de A₁. L'ordonnée Q₁ de B₁ donne la quantité de principe actif contenue à l'instant de la mesure dans le réservoir suivant l'invention.

Des mesures de concentration plasmatique, opérées in vivo sur un patient en cours de traitement, par des moyens connus, chromatographiques ou electrochimiques par exemple, permettraient également d'établir l'abaque de la figure 3.

Suivant une caractéristique avantageuse du dispositif selon l'invention, le microcontrôleur 6 comprend des moyens de mémoire (non représentés) chargés avec une table, tirée de l'abaque de la figure 3, faisant correspondre à une suite de valeurs de la tension V, une suite de valeurs de la quantité Q de principe actif contenu dans le réservoir. Grâce à la connaissance, à tout instant, de cette quantité Q, le microcontrôleur élabore un signal de contre-réaction utilisable dans la commande du courant thérapeutique par le microcontrôleur 6, pour contraindre l'intensité de ce courant à suivre un programme temporel prédéterminé, correspondant à un programme d'administration de médicament prédéterminé.

On remarquera que la commande ainsi établie de ce courant permet de tenir compte des variations du rendement ionophorétique, d'un patient à l'autre, et des variations dans le temps de la perméabilité au principe actif de la peau d'un patient.

Bien entendu l'invention n'est pas limitée au mode de réalisation décrit et représenté qui n'a été donné qu'à titre d'exemple. Ainsi, en période de mesure de la quantité de principe actif passée dans le sang du patient, on peut utiliser un courant d'intensité différente de celle du courant thérapeutique. On peut alors procéder à la mesure dans un domaine courant-tension choisi de manière à soustraire la mesure à d'éventuelles perturbations d'origine physiologique.

De même, le réservoir peut comprendre une électrode 10 de mesure prenant une forme autre que celle d'une grille métallique, par exemple celle d'un film ajouré et plaqué avec une couche métallique, ou même un film ajouré en un matériau polymère conducteur de l'électricité.

La couche d'hydrogel 12 qui recouvre la face externe de l'électrode de mesure 10 a plusieurs fonctions. Elle sert d'abord à assurer l'adhérence de l'électrode de mesure sur la peau d'un patient tout en atténuant l'irritation de celle-ci. Elle sert aussi alors à isoler l'électrode 10 d'ions tels que K⁺, Na⁺, Cl⁻ normalement présents sur la peau du patient du fait de la transpiration. Elle sert encore à protéger l'électrode de mesure lorsque le réservoir n'est pas appliqué sur la peau d'un patient.

Le polymère hydratable ou hydrogel constituant la couche 2 peut être remplacé par d'autres matériaux tels qu'un feutre, une éponge ou tout autre matériau susceptible d'absorber un liquide.

Il est clair aussi que l'invention s'étend à la détermination de la quantité de principe actif sous forme ionisée passé dans le sang d'un patient par administration transdermique, quand bien même celle-ci ne serait pas assistée par ionophorèse. Cependant, elle s'adapte particulièrement facilement à un dispositif ionophorétique du fait de la présence dans celui-ci de l'essentiel des moyens électriques et électroniques nécessaires.

## Revendications

1. Procédé de mesure de la quantité d'un principe actif contenu sous une forme ionisée dans un réservoir (1) constitué par une couche (2) d'un matériau imprégné d'une solution de ce principe actif, caractérisé en ce qu'on mesure la conductivité du matériau imprégné et on tire une mesure de la quantité de principe actif contenu dans le réservoir de cette mesure de conductivité.

2. Procédé conforme à la revendication 1, caractérisé en ce que, pour mesurer la conductivité du matériau imprégné, on fait passer un courant d'intensité (I) prédéterminée dans celui-ci, et on mesure la tension électrique (V) qui apparaît alors entre deux électrodes (7,10) séparées par au moins une partie de la couche (2) du matériau imprégné .

3. Procédé conforme à la revendication 2, caractérisé en ce qu'on tire la quantité (Q) de principe actif contenu dans le réservoir d'une abaque établie lors d'une phase d'étalonnage préalable et donnant cette quantité (Q) en fonction de ladite tension mesurée.

4. Procédé conforme à la revendication 3, caractérisé en ce qu'on établit ladite abaque à partir de mesures de quantités (Q) de principe actif ayant traversées un échantillon de peau sous administration transdermique du principe actif assistée par ionophorèse, opérées dans une cellule de mesures in vitro.

5. Procédé conforme à la revendication 3, caractérisé en ce qu'on établit ladite abaque à partir de mesures de quantités (Q) de principe actif ayant traversées la peau d'un patient.

6. Dispositif pour la mise en oeuvre du procédé conforme à la revendication 1, caractérisé en ce qu'il comprend a) des première (7) et deuxième (10) électrodes placées de part et d'autre et en contact électrique avec au moins une partie de la couche du matériau imprégné du réservoir, b) des moyens (5) pour faire passer le courant électrique d'intensité prédéterminée dans le matériau imprégné et c) des moyens (6) sensibles à la tension (V) relevée entre les électrodes (7,10) pour calculer la quantité de principe actif contenu dans le réservoir.

7. Dispositif conforme à la revendication 6, caractérisé en ce que lesdits moyens de calcul (6) sont incorporés à un module électronique (4) de génération et de commande d'un courant électrique thérapeutique d'administration transdermique du principe actif, assistée par ionophorèse.

8. Dispositif conforme à la revendication 7, caractérisé en ce que les moyens pour faire passer le courant électrique dans le matériau imprégné comprennent un générateur (5) débitant un courant passant entre la première électrode (7) et la deuxième électrode (10), cette dernière étant en contact électrique avec la peau d'un patient.

9. Dispositif conforme à la revendication 6 pour la mise en oeuvre du procédé conforme à la revendication 3, caractérisé en ce que les moyens de calcul (6) comprennent des moyens de mémoire chargés avec une table donnant la quantité de principe actif contenu dans le réservoir en fonction de la tension électrique mesurée entre les électrodes, la table constituant une image de ladite abaque.

10. Dispositif conforme à la revendication 9, caractérisé en ce qu'il comprend des moyens de régulation d'un courant thérapeutique à partir de la quantité (Q) de principe actif mesurée, de manière à asservir la quantité de principe actif administrée à un patient à un programme temporel prédéterminé.

11. Réservoir pour la mise en oeuvre du procédé conforme à l'une quelconque des revendications 1 à 5, comportant une couche (2) d'un matériau susceptible d'être imprégné d'une solution du principe actif en vue d'une administration transdermique de ce principe actif à un patient, à travers une face de cette couche appliquée contre la peau du patient, caractérisé en ce qu'il comprend une électrode (10) de mesure perméable à la solution de principe actif, appliquée sur ladite face de la couche (2) du matériau imprégnable.

12. Réservoir conforme a la revendication 11, caractérisé en ce que ladite électrode (10) de mesure prend la forme d'une grille conductrice.

13. Réservoir conforme à la revendication 12, caractérisé en ce que l'électrode de mesure (10) est recouverte, du côté qui n'est pas en contact avec la couche (2) du matériau imprégnable, avec une couche (12) d'un hydrogel neutre.

14. Réservoir conforme à l'une quelconque des revendications 11 à 13, caractérisé en ce qu'il comprend une électrode (7), disposée en contact électrique avec la face de la couche (2) de matériau imprégnable qui est opposée à celle en contact avec l'électrode (10) de mesure.

## Claims

1. Method for measuring the quantity of an active principle contained in an ionized form in a reservoir (1) consisting of a layer (2) of a material impregnated with a solution of this active principle, characterized in that the conductivity of the impregnated material is measured and a measurement of the quantity of active principle contained in the reservoir is derived from this conductivity measurement.

2. Method according to Claim 1, characterized in that, in order to measure the conductivity of the impregnated material, a current of predetermined strength (I) is passed through the latter, and the electric voltage (V) which then appears between two electrodes (7, 10) separated by at least a part of the layer (2) of the impregnated material is measured.

3. Method according to Claim 2, characterized in that the quantity (Q) of active principle contained in the reservoir is derived from a plot established during a prior calibration phase and giving this quantity (Q) as a function of the said measured voltage.

4. Method according to Claim 3, characterized in that the said plot is established on the basis of measurements of quantities (Q) of active principle which have passed through a skin sample under transdermal delivery of the active principle assisted by iontophoresis, carried out in an in vitro measurement cell.

5. Method according to Claim 3, characterized in that the said plot is established on the basis of measurements of quantities (Q) of active principle which have passed through the skin of a patient.

6. Device for implementing the method according to Claim 1, characterized in that it comprises a) first (7) and second (10) electrodes placed on either side of and in electrical contact with at least a part of the layer of the impregnated material of the reservoir, b) means (5) for passing the electric current of predetermined strength through the impregnated material and c) means (6) sensitive to the voltage (V) picked up between the electrodes (7, 10) in order to calculate the quantity of active principle contained in the reservoir.

7. Device according to Claim 6, characterized in that the said calculation means (6) are incorporated in an electronic module (4) for generating and controlling a therapeutic electric current for transdermal delivery of the active principle, assisted by iontophoresis.

8. Device according to Claim 7, characterized in that the means for passing the electric current through the impregnated material comprise a generator (5) delivering a current passing between the first electrode (7) and the second electrode (10), the latter being in electrical contact with the skin of a patient.

9. Device according to Claim 6 for implementing the method according to Claim 3, characterized in that the calculation means (6) comprise memory means loaded with a table giving the quantity of active principle contained in the reservoir as a function of the electric voltage measured between the electrodes, the table constituting an image of the said plot.

10. Device according to Claim 9, characterized in that it comprises means for regulating a therapeutic current on the basis of the measured quantity (Q) of active principle, so as to slave the quantity of active principle delivered to a patient to a predetermined time programme.

11. Reservoir for implementing the method according to Claim 1, comprising a layer (2) of a material which can be impregnated with a solution of the active principle with a view to transdermal delivery of this active principle to a patient, through a face of this layer which is applied against the skin of the patient, characterized in that it comprises a measurement electrode (10) permeable to the solution of active principle, applied to the said face of the layer (2) of the impregnatable material.

12. Reservoir according to Claim 11, characterized in that the said measurement electrode (10) is in the form of a conductive grid.

13. Reservoir according to Claim 12, characterized in that the measurement electrode (10) is covered, on the side which is not in contact with the layer (2) of the impregnatable material, with a layer (12) of a neutral hydrogel.

14. Reservoir according to Claim 11, characterized in that it comprises an electrode (7), arranged in electrical contact with that face of the layer (2) of impregnatable material which is opposite the face in contact with the measurement electrode (10).

## Patentansprüche

1. Verfahren zum Messen der Menge eines Wirkstoffs, der in einer ionisierten Form in einem Behälter (1) enthalten ist, der aus einer Schicht (2) aus einem mit einer Lösung dieses Wirkstoffs getränkten Material besteht, dadurch gekennzeichnet, daß man die spezifische elektrische Leitfähigkeit des getränkten Materials mißt und aus dieser Leitfähigkeitsmessung eine Messung der Menge des im Behälter enthaltenen Wirkstoffs ableitet.

2. Verfahren nach Anspruch 1, dadurch gekennzeichnet, daß man zum Messen der Leitfähigkeit des getränkten Materials in diesem einen Strom mit einer vorbestimmten Stromstärke (I) fließen läßt und die elektrische Spannung (V) mißt, die dabei zwischen zwei Elektroden (7, 10) auftritt, die mindestens durch einen Teil der Schicht (2) aus getränktem Material getrennt sind.

3. Verfahren nach Anspruch 2, dadurch gekennzeichnet, daß man die Menge (Q) des im Behälter enthaltenen Wirkstoffs aus einer in einer vorhergehenden Eichphase erstellten Rechentafel zieht, die diese Menge (Q) in Abhängigkeit von der gemessenen Spannung angibt.

4. Verfahren nach Anspruch 3, dadurch gekennzeichnet, daß man die Rechentafel ausgehend von in einer In-vitro-Meßzelle vorgenommenen Messungen von Wirkstoffmengen (Q) erstellt, die eine Hautprobe unter iontophoresegestützter transdermaler Anwendung des Wirkstoffs durchquert haben.

5. Verfahren nach Anspruch 3, dadurch gekennzeichnet, daß man die Rechentafel ausgehend von Messungen von Wirkstoffmengen (Q) erstellt, die die Haut eines Patienten durchquert haben.

6. Vorrichtung zur Durchführung des Verfahrens nach Anspruch 1, dadurch gekennzeichnet, daß sie aufweist:
a) eine erste Elektrode (7) und eine zweite Elektrode (10), die zu beiden Seiten und in elektrischem Kontakt mit mindestens einem Teil der Schicht aus getränktem Material des Behälters angeordnet sind,
b) Einrichtungen (5), um den elektrischen Strom mit vorbestimmter Stromstärke in dem getränkten Material fließen zu lassen, und
c) Einrichtungen (6), die gegenüber der zwischen den Elektroden (7, 10) erfaßten Spannung (V) empfindlich sind, um die Menge des im Behälter enthaltenen Wirkstoffs zu **berechnen.**

7. Vorrichtung nach Anspruch 6, dadurch gekennzeichnet, daß die Recheneinrichtungen (6) einem elektronischen Modul (4) zur Erzeugung und zur Steuerung eines therapeutischen elektrischen Stroms für die iontophoresegestützte transdermale Anwendung des Wirkstoffs eingegliedert sind.

8. Vorrichtung nach Anspruch 7, dadurch gekennzeichnet, daß die Einrichtungen, um den elektrischen Strom in dem getränkten Material fließen zu lassen, einen Generator (5) umfassen, der einen Strom abgibt, der zwischen der ersten Elektrode (7) und der zweiten Elektrode (10) fließt, wobei letztere mit der Haut eines Patienten in Kontakt ist.

9. Vorrichtung nach Anspruch 6 zur Durchführung des Verfahrens nach Anspruch 3, dadurch gekennzeichnet, daß die Recheneinrichtungen (6) Speichereinrichtungen umfassen, die mit einer Tabelle geladen sind, die die Menge des im Behälter enthaltenen Wirkstoffs in Abhängigkeit von der zwischen den Elektroden gemessenen elektrischen Spannung angibt, wobei die Tabelle ein Bild der Rechentafel darstellt.

10. Vorrichtung nach Anspruch 9, dadurch gekennzeichnet, daß sie Einrichtungen zur Regulierung eines therapeutischen Stroms ausgehend von der gemessenen Wirkstoffmenge (Q) aufweist, so daß die einem Patienten verabreichte Wirkstoffmenge nach einem vorbestimmten zeitlichen Programm gesteuert wird.

11. Behälter zur Durchführung des Verfahrens nach einem der Ansprüche 1 bis 5, der eine Schicht (2) aus einem Material umfaßt, das mit einer Lösung des Wirkstoffs getränkt werden kann, und zwar zum Zweck einer transdermalen Verabreichung dieses Wirkstoffs an einen Patienten durch eine an die Haut des Patienten angelegte Seite dieser Schicht hindurch, dadurch gekennzeichnet, daß er eine für die Wirkstofflösung durchlässige Meßelektrode (10) aufweist, die an diese Seite der Schicht (2) aus tränkbarem Material angelegt ist.

12. Behälter nach Anspruch 11, dadurch gekennzeichnet, daß die Meßelektrode (10) die Form eines leitenden Gitters hat.

13. Behälter nach Anspruch 12, dadurch gekennzeichnet, daß die Meßelektrode (10) auf der Seite, die nicht mit der Schicht (2) aus tränkbarem Material in Kontakt ist, mit einer Schicht (12) aus einem neutralen Hydrogel bedeckt ist.

14. Behälter nach einem der Ansprüche 11 bis 13, dadurch gekennzeichnet, daß er eine Elektrode (7) aufweist, die in elektrischem Kontakt mit der Seite der Schicht (2) aus tränkbarem Material angeordnet ist, die der mit der Meßelektrode (10) in Kontakt befindlichen Seite entgegengesetzt ist.
